(19) Europäisches Patentamt
European Patent Office
Office européen des brevets

(11) **EP 4 355 915 B1**

(12) **EUROPEAN PATENT SPECIFICATION**

(45) Date of publication and mention of the grant of the patent:
**04.02.2026 Bulletin 2026/06**

(21) Application number: **22734284.7**

(22) Date of filing: **15.06.2022**

(51) International Patent Classification (IPC):
***C12Q 1/6883*** *(2018.01)* ***A61P 1/16*** *(2006.01)*

(52) Cooperative Patent Classification (CPC):
**C12Q 1/6883; A61P 1/16;** C12Q 2600/106; C12Q 2600/112; C12Q 2600/178

(86) International application number:
**PCT/EP2022/066410**

(87) International publication number:
**WO 2022/263565 (22.12.2022 Gazette 2022/51)**

(54) **METHOD FOR NASH RISK ASSESSMENT IN PATIENTS HAVING A METABOLIC DISORDER**

VERFAHREN ZUR NASH-RISIKOBEWERTUNG BEI PATIENTEN MIT EINER STOFFWECHSELSTÖRUNG

PROCÉDÉ D'ÉVALUATION DE RISQUE NASH CHEZ DES PATIENTS SOUFFRANT D'UN TROUBLE MÉTABOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2021 EP 21305826**

(43) Date of publication of application:
**24.04.2024 Bulletin 2024/17**

(73) Proprietor: **Genfit**
**59120 Loos (FR)**

(72) Inventors:
- **HAJJI, Yacine**
  **59000 Lille (FR)**
- **MAGNANENSI, Jérémy**
  **59800 Lille (FR)**
- **ROSENQUIST, Christian**
  **92200 Neuilly-sur-Seine (FR)**
- **HOSMANE, Suneil**
  **Arlington, MA 02474 (US)**

(74) Representative: **Cabinet Becker et Associés**
**25, rue Louis le Grand**
**75002 Paris (FR)**

(56) References cited:
**WO-A1-2017/167934 WO-A1-2022/090452**

- **KOGACHI SHANNON ET AL: "Noninvasive Evaluation for Nonalcoholic Fatty Liver Disease and Nonalcoholic Steatohepatitis", CLINICAL THERAPEUTICS, ELSEVIER, AMSTERDAM, NL, vol. 43, no. 3, 10 February 2021 (2021-02-10), pages 455 - 472, XP086540492, ISSN: 0149-2918, [retrieved on 20210210], DOI: 10.1016/J.CLINTHERA.2021.01.012**
- **HARRISON STEPHEN A ET AL: "A blood-based biomarker panel (NIS4) for non-invasive diagnosis of non-alcoholic steatohepatitis and liver fibrosis: a prospective derivation and global validation study", vol. 5, no. 11, 1 November 2020 (2020-11-01), US, pages 970 - 985, XP055792366, ISSN: 2468-1253, Retrieved from the Internet <URL:http://dx.doi.org/10.1016/S2468-1253(20)30252-1> DOI: 10.1016/S2468-1253(20)30252-1**
- **"Late breaker posters ED - Dufour Jean-François; Schwabe Robert F; Terrault Norah; Wong Vincent", JOURNAL OF HEPATOLOGY, ELSEVIER, AMSTERDAM, NL, vol. 75, 25 June 2021 (2021-06-25), XP086676458, ISSN: 0168-8278, [retrieved on 20210625], DOI: 10.1016/S0168-8278(21)01843-2**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 355 915 B1

## Description

### FIELD OF THE INVENTION

The present invention relates to a new assay to diagnose at-risk nonalcoholic steatohepatitis (NASH) in patients having a metabolic disorder.

### BACKGROUND OF THE INVENTION

NASH is a progressive disease of the liver characterized histologically by fatty acid accumulation, hepatocyte damage and inflammation resembling alcoholic hepatitis. NASH can lead to liver fibrosis, cirrhosis, liver failure and/or hepatocellular carcinoma (HCC). Along with the obesity and type-2 diabetes rates in the world, the incidence of NASH has increased in recent years, and patients who develop NASH have an increased rate of liver-related mortalities. In particular, patients with metabolic disorders, such as type 2 diabetes (T2D), are at risk of having NASH and cirrhosis (Caldazilla S.H. et al, Int J Mol Sci, 2016,17(5):774). More particularly, T2D is an independent risk factor for the development of NASH (Tomah, S. et al, Clinical Diabetes and Endocrinology, 2020, 6: 9).

KOGACHI SHANNON ET AL: "Noninvasive Evaluation for Nonalcoholic Fatty Liver Disease and Nonalcoholic Steatohepatitis",CLINICAL THERAPEUTICS, vol. 43, no. 3, 10 February 2021, pages 455-472 describes the combination of two marker sets (NFS and FIB-4) for diagnosing the risk of NASH in a patient.

NASH remains largely underdiagnosed, as it carries no obvious symptoms in its early stages, and also because of the lack of widely available non-invasive tests specifically developed to diagnose the disease. The underdiagnosis of NASH represents a major unmet need and is a barrier for identification of patients at higher risk for disease progression, like diabetic patients, who would be eligible for therapeutic intervention.

Sequential use of blood-based diagnostic tests has the potential to improve the overall diagnostic accuracy of identifying at-risk NASH. Sensitivity (the ability of a test to correctly identify those who have a disease) and specificity (the ability of a test to correctly identify those who do not have a disease) are two essential indicators of test accuracy and allow healthcare providers to determine the appropriateness of the diagnostic tool (Glaros A.G. et al, J Clin Psychol, 1988, 44(6):1013-23). However, sensitivity and specificity are inversely related: as sensitivity increases, specificity tends to decrease. There is therefore a need to identify the most accurate and balanced performing blood-based testing algorithms having the potential to detect at-risk NASH in patients suffering from a metabolic disorder, such as in T2D patients.

### SUMMARY OF INVENTION

The inventors herein show that a combination of two different noninvasive tests, namely NIS4 and NFS, provides the most accurate and balanced diagnostic of at-risk NASH in patients with a metabolic disorder, such as T2D.

Therefore, the invention relates to a method for the diagnosis of at-risk NASH in a human patient with a metabolic disorder, comprising:

(i) calculating a nonalcoholic fatty liver disease score (NFS) from the age, body-mass index, aspartate amino transferase (AST)/ alanine aminotransferase (ALT) ratio, platelet count and hyperglycemia status of said patient; and
(ii) calculating a nonalcoholic steatohepatitis score (NIS4) for said patient, based on the level of hsa-miR-34a-5p, alpha-2 macroglobulin (A2M), YKL-40 and glycated haemoglobin (HbA1c) measured in a blood or blood-derived sample of said patient;

and determining the presence or absence of at-risk NASH in said patient based on said scores;
wherein the NFS test is implemented before or after the NIS4 test.

In some embodiments, the calculated NFS is compared to a NFS low cutoff value and the calculated NIS4 is compared to a NIS4 low cutoff value. In other embodiments, if the calculated NFS is lower than said NFS low cutoff value or if the calculated NIS4 is lower than said NIS4 low cutoff NIS4, then the patient is diagnosed as not having at-risk NASH. In other embodiments, if the calculated NFS is greater or equal to said NFS low cutoff value and if the calculated NIS4 is greater or equal to said NIS4 low cutoff value, then the patient is diagnosed as having at-risk NASH. In a particular embodiment, said NFS low cutoff value is equal to -1.455. In another particular embodiment, said NIS4 low cutoff value is equal to 0.36.

The invention further relates to an anti-NASH or anti-fibrotic agent for use in a method for the treatment of NASH in a patient in need thereof diagnosed as having at-risk NASH according to the method of diagnosis disclosed herein. The invention further relates to an anti-NASH or anti-fibrotic agent for use in a method for slowing down or stopping the progression of NASH in a patient in need thereof diagnosed as having at-risk NASH according to the method of diagnosis

disclosed herein.

## BRIEF DESCRIPTION OF THE DRAWINGS

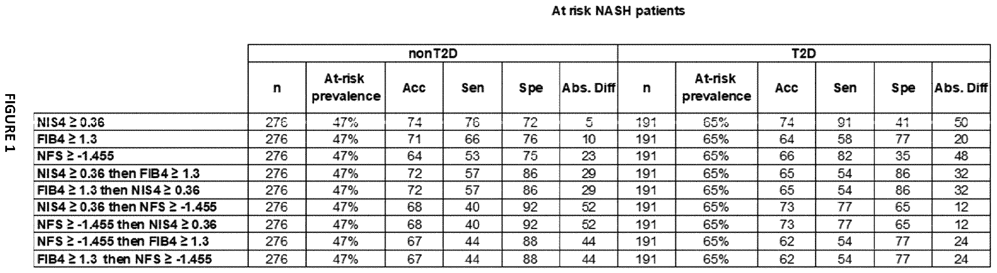

Figure 1 shows the diagnostic performances of individual and sequential scores within non-Type 2 diabetic (non-T2D) and Type 2 diabetic (T2D) patients within the Resolve-IT study. The scores have been evaluated according to the different methods (NIS4 test, FIB4 test and NFS test in combination or not).
n: number of patients enrolled in the study; acc: accuracy; sen: sensitivity; spe: specificity; abs. diff= absolute difference between sensitivity and specificity.

## DETAILED DESCRIPTION OF THE INVENTION

NASH and fibrosis scoring/staging

Histological scoring/staging systems have been developed to assess NAFLD activity level and fibrosis stage and to estimate the risk of evolution to clinical liver outcomes. The NALFD-Activity-Score (NAS) has been developed to assess the severity of NAFLD. NAS is the sum of three histological scores determined from liver biopsy slices:

- S: Steatosis score: 0: <5%; 1: 5-33%; 2: 34-66% and 3: >66%;
- LI: Lobular Inflammation score (foci per 20x field): 0: none; 1: <2 foci; 2: 2-4 foci and 3: >4 foci; and
- HB: Ballooning degeneration score: 0: none; 1: few; 2: many cells/prominent ballooning.

Using this scoring system, a "patient with NASH" has NAS≥3, with at least 1 point in steatosis, at least 1 point in lobular inflammation and at least 1 point in hepatocyte ballooning. A "non-NASH" patient is a patient having either (i) a NAS≥3 with at least one of steatosis, lobular inflammation and hepatocyte ballooning scores equal to 0; or (ii) a NAS<3. In addition, in the context of the present invention, a patient is excluded as being a NASH patient if said patient has viral hepatitis, alcohol-related liver disease, autoimmune liver disease, drug-induced liver disease or congenital causes of chronic liver disease such as hereditary hemochromatosis, Wilson's disease, alpha-1-antitrypsin deficiency and polycystic ovary syndrome.

Localization and extent of fibrosis (F) at histological exam signs the severity (advancement) of NASH. The NASH-CRN (Nonalcoholic SteatoHepatitis Clinical Research Network) has developed a dedicated fibrosis staging system (Kleiner, D.E et al, Hepatology, 2005 Jun; 41(6):1313-21).

| NASH Clinical Research Network Scoring System Definitions | F Score |
|---|---|
| Perisinusoidal or periportal fibrosis | 1 |
| Mild perisinusoidal fibrosis (zone 3) | 1a |
| Moderate perisinusoidal fibrosis (zone 3) | 1b |
| Portal/periportal fibrosis | 1c |
| Perisinusoidal and portal/periportal fibrosis | 2 |
| Bridging fibrosis | 3 |
| Cirrhosis | 4 |

Using this fibrosis staging system, patients with no or minimal fibrosis (F=0-1) are generally not considered at risk of cirrhosis, liver failure, HCC (hepatocellular carcinoma) or liver-related death. Patients with significant (F=2) and moderate fibrosis (F=3) are at increasing risk of developing cirrhosis, liver failure, HCC and liver-related death. Patients with compensated cirrhosis have severe fibrosis (F=4) and are at high risk of liver failure (decompensated cirrhosis), HCC and liver-related deaths. Identifying patients who are at risk of developing HCC, cirrhotic complications and liver-related deaths is the ultimate reason for liver assessment. As defined by the FDA and EMA, patients at risk of liver outcomes who should be pharmacologically treated are those with NAS≥4 (with score ≥ 1 for each of steatosis, lobular inflammation and ballooning) and NASH-CRN fibrosis score (F) ≥ 2.

Accordingly, in the context of the present invention, a patient with "at-risk NASH", otherwise referred to as an "at-risk patient" or as a "patient at risk of hepatic outcome", is a patient with a NAS higher or equal to 4, a S score higher or equal to 1, a LI score higher or equal to 1, a HB score higher or equal to 1 and a F score of 2. It defines a subgroup of NASH patients having a high risk of developing at least one life-threatening liver outcome such as cirrhosis, liver failure, HCC and liver-related death.

The present invention relates to improved methods useful to detect at-risk NASH in patients with a metabolic disorder, such as T2D patients.

Method of the invention

It is herein shown that the combination of two noninvasive tests, NFS and NIS4, provides the most accurate and balanced diagnosis of at-risk NASH in patients with a metabolic disorder, in particular in patients with T2D. Indeed, advanced forms of NASH occur more commonly in patients with metabolic disorders, such as in T2D patients. As mentioned above, NASH increases the risk of cirrhosis, liver failure and HCC. Detection of at-risk NASH in such patients is thus of outmost importance.

Furthermore, it is also important to have a method that allows the most confident decision-making, with the lowest possible percentage of patients falling in the indeterminate category, and the highest possible percentage of patients most confidently classified as having a high or low probability of at-risk NASH.

The present invention provides a solution to these unmet needs.

The inventors have shown that NIS4 is the most accurate and balanced test to detect subjects with at-risk NASH among subjects who do not have T2D, as compared to other noninvasive tests. They have further shown that from T2D patient samples, combining both NFS and NIS4 resulted in a more accurate and balanced detection of subjects with at-risk NASH, with a high sensitivity of 77% and a high specificity of 65%.

The present invention, which is directed to a method combining both NFS and NIS4, is thus particularly advantageous for the early detection of at-risk NASH in patients with metabolic disorders such as T2D patients.

*Patients in need of the method of the invention*

As mentioned above, NASH occurs more commonly in patients suffering from metabolic disorders. In addition, NASH is known to be associated to comorbidities such as metabolic disorders. Therefore, the method of the present invention can benefit to those patients presenting such comorbidities.

Common comorbidities of NASH include obesity, insulin resistance, glucose intolerance, T2D, prediabetes, dyslipidaemia, hypertriglyceridaemia, hypertension and cardiovascular disease. Older age may also predispose to HCC in NASH patients.

In a particular embodiment, the patient suffers from a metabolic disorder, such as obesity, insulin resistance, glucose intolerance, T2D, prediabetes, dyslipidaemia and hypertriglyceridaemia.

In a preferred embodiment, the patient is a T2D patient.

*NFS test*

The "nonalcoholic fatty liver disease fibrosis score", or "NFS", is a noninvasive diagnosis test that was developed to detect advanced liver fibrosis (i.e. fibrosis stage F $\geq$ 3) in subjects with NAFLD (Angulo et al., Hepatology, 2007;45:846-854). It is based on age, BMI, AST/ALT ratio, platelet count and hyperglycemia (or IFG/diabetes status: yes or no). The score has 2 cutoff values: a score lower than -1.455 predicts the absence of advanced fibrosis, whereas a score greater than 0.675 predicts the presence of advanced fibrosis. The NFS has been validated in multiple studies. NAFLD guidelines acknowledged that the NFS is a clinically useful tool for identifying advanced fibrosis in patients with NAFLD. This score is available online at http://nafldscore.com/, and it can be easily calculated during patient visits. However, a major drawback of this score is that a large percentage of patients fall in the indeterminate category and cannot be classified as having a high or low probability of advanced fibrosis.

In the context of the present invention, the method for detecting at-risk NASH implements the use of the NFS test, as described above and in Angulo *et al.,* 2007.

In another particular embodiment, the score obtained is compared to a low cutoff value, such as the low cutoff value of -1.455 disclosed in Angulo *et al.,* 2007. In the context of the present invention, if the calculated NFS is:

- lower than the low cutoff value, then the patient is classified as not having at-risk NASH;
- greater or equal to the low cutoff value, then a NIS4 test is implemented.

*NIS4 test*

"NIS4" denotes a blood-based diagnostic test to identify at-risk NASH. NIS4 is based on the measure of the level of four circulating biomarkers: hsa-miR-34a-5p, alpha-2 macroglobulin (A2M), YKL-40 (also known as chitinase 3-like protein 1 [CHI3L1]) and glycated haemoglobin (HbA1c). This test is described in WO2017167934 and in Harrison et al, The

Lancet Gastroenterology and Hepatology, 2020; 5(11):P970-985.

The present invention thus implements the measure of the level of hsa-miR-34a-5p, A2M, YKL40 and Hb1Ac in a blood-derived sample of a patient suffering from a metabolic disorder, such as a T2D patient. In a particular embodiment, the blood-derived sample may be blood, serum or plasma, in particular platelet-free plasma, e.g. a cell-free, citrate-derived platelet-free plasma sample. In a particular embodiment, the level of hsa-miR-34a-5p, A2M and YKL40 is measured from one or more serum sample(s) from the subject. In another particular embodiment, the level of HbA1c is measured from a blood sample of the subject. In a particular embodiment, the level of hsa-miR-34a-5p, A2M, YKL40 and HbA1c is measured as described in WO2017167934.

In a particular embodiment, the levels of the circulating markers as measured herein are used in a logistic function to calculate a score, for example as provided in application WO2017167934 and in Harrison *et al.* 2020.

One skilled in the art can use the information provided in WO2017167934 and Harrison *et al.* 2020 to determine the logistic function and cutoffs relevant to the required test sensitivity, specificity, positive predictive value and/or negative predictive value.

In a particular embodiment, the NIS4 score is calculated, and can then be compared to a low cutoff value as described below.

The NIS4 score is more particularly calculated as provided in WO2017167934 and Harrison *et al,* 2020, as follows:

$$S = \frac{e^{Y}}{1 + e^{Y}}$$

wherein:

$$Y=k+a*A+b*B+c*C+d*D$$

wherein:

S is the NIS4 score;
A is the serum level of hsa-miR-34a-5p in Cq;
B is the serum level of alpha 2 macroglobulin in g/L;
C is the serum level of YKL-40 in ng/mL,
D is the level of HbA1c in percent (e.g. D is equal to 10 if measured HbA1c percentage is 10%);
k is the constant of the logistic function
a is a coefficient associated to the serum level of hsa-miR-34a-5p;
b is a coefficient associated to the serum level of alpha 2 macroglobulin;
c is a coefficient associated to the serum level of YKL-40; and
d is a coefficient associated to the level of HbA1c.

In a particular embodiment, derived from NIS4 score as described in this application:

k is a number comprised between 9.51 and 34.37;
a is a number comprised between -1.17 and -0.47;
b is a number comprised between 0.02 and 0.84;
c is a number comprised between 0.0061 and 0.0209; and
d is a number comprised between 0.07 and 0.89;
and wherein the low cutoff value is comprised between 0.2013 and 0.5965, and is more particularly equal to 0.36.

In a further particular embodiment, derived from NIS4 score as described in this application:

k is a number comprised between 12,24 and 22,74;
a is a number comprised between -0.93 and -0.5;
b is a number comprised between 0.36 and 0.68;
c is a number comprised between 0.005 and 0.015;
d is a number comprised between 0.37 and 0.69;
and wherein the low cutoff value is comprised between 0.2013 and 0.5965, and is more particularly equal to 0.36.

In a particular embodiment, the calculated NIS4 score is compared to a low cutoff value of 0.36, as disclosed in

Harrison *et al.,* 2020.

According to the present invention, the NIS4 test is implemented before or after the NFS test. Of note, if the NFS is implemented first, the NIS4 test is implemented after the NFS test if the latter provides a calculated score greater or equal to a low cutoff NFS value. Otherwise, if the NFS test is implemented first and provides a calculated score lower than a low cutoff NFS value, then the NIS4 test is not implemented.

The calculated NIS4 is compared to a low cutoff value (e.g., a low cutoff value with a sensitivity >80% and balanced specificity) established to minimize false negatives and enable a confident rule-out diagnosis. In a particular embodiment, as mentioned above, said low cutoff value is selected in the range comprised between 0.2013 and 0.5965. In a more particular embodiment, said low cutoff value is equal to 0.36.

In the context of the embodiment wherein the NIS4 test is implemented after the NFS test, (i) if the calculated NIS4 is lower than the low cutoff value, then the patient is classified as having a low probability to have at-risk NASH, or as not having at-risk NASH and (ii) if the calculated NIS4 is greater or equal to the low cutoff value, then the patient is classified as having a high probability to have at-risk NASH, or as having at-risk NASH.

In the context of the embodiment wherein the NIS4 test is implemented before the NFS test, if the calculated NIS4 is:

- lower than the low cutoff value, then the patient is classified as not having at-risk NASH;
- greater or equal to the low cutoff value, then a NFS test is implemented.

In some embodiments, thanks to the method of the invention, a decision may be taken to give life style recommendations to a patient (such as a food regimen or providing physical activity recommendations), to medically take care of a patient (e.g. by setting regular visits to a physician or regular examinations, for example for regularly monitoring markers of liver damage), or to administer at least one NASH or liver fibrosis therapy to the patient, to treat or prevent at-risk NASH. In a particular embodiment, a decision may be taken to give life style recommendations to a subject or to administer at least one NASH or liver fibrosis therapy.

The invention thus further relates to an anti-NASH or anti-fibrotic compound for use in a method for treating NASH or liver fibrosis in a subject in need thereof, wherein the subject has been identified thanks to a method according to the invention.

The term "treatment", as used herein, relates to both therapeutic measures and prophylactic or preventive measures, wherein the goal is to prevent or slow down (lessen) an undesired physiological change or disorder. Beneficial or desired clinical results include, but are not limited to, alleviation of symptoms, stabilizing pathological state (specifically not worsening), slowing down or stopping the progression of the disease, improving or mitigating the pathological condition. Particularly, for the purpose of the present invention, treatment is directed to slow the progression of sepsis and reduce the risk of further complications. It can also involve prolonging survival in comparison with the expected survival if the treatment is not received.

The anti-NASH or anti-fibrotic agent is administered in a therapeutically effective amount. As used herein, the expression "therapeutically effective amount" refers to an amount of the drug effective to achieve a desired therapeutic result. A therapeutically effective amount of a drug may vary according to factors such as the disease state, age, sex, and weight of the individual, and the ability of drug to elicit a desired response in the individual. A therapeutically effective amount is also one in which any toxic or detrimental effects of agent are outweighed by the therapeutically beneficial effects. The effective dosages and dosage regimens for drug depend on the disease or condition to be treated and may be determined by the persons skilled in the art. A physician having ordinary skill in the art may readily determine and prescribe the effective amount of the pharmaceutical composition required. For example, the physician could start doses of drug employed in the pharmaceutical composition at levels lower than that required in order to achieve the desired therapeutic effect and gradually increase the dosage until the desired effect is achieved. In general, a suitable dose of a composition of the present invention will be that amount of the compound which is the lowest dose effective to produce a therapeutic effect according to a particular dosage regimen. Such an effective dose will generally depend upon the factors described above.

In a particular embodiment, the invention relates to an anti-NASH compound for use in a method for treating NASH in a patient suffering from a metabolic disorder and at-risk NASH, wherein the patient has been classified as having at-risk NASH thanks to the method according to the invention. In another particular embodiment, the invention relates to an anti-NASH compound for use in a method for treating NASH in a T2D patient with at-risk NASH, wherein the T2D patient has been classified as having at-risk NASH thanks to the method according to the invention.

The invention further relates to an anti-fibrotic compound for use in a method for treating liver fibrosis in a patient suffering from a metabolic disorder and at-risk NASH, wherein the patient has been classified as a receiver of said treatment thanks to a method according to the invention. The invention also relates to an anti-fibrotic compound for use in a method for treating liver fibrosis in a T2D patient with at-risk NASH, wherein the T2D patient has been classified as a receiver of said treatment thanks to a method according to the invention.

Illustrative anti-NASH and anti-fibrotic compounds are listed below:

- a compound of formula (I) or a pharmaceutically acceptable salt thereof:

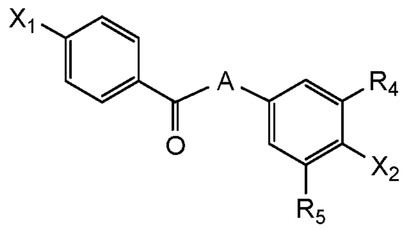

(I)

wherein:

X1 represents a halogen atom, a R1 group or G1-R1 group;
A represents a CH=CH or CH2-CH2 group;
X2 represents a G2-R2 group;
G1 and G2, identical or different, represent an atom of oxygen or sulfur;
R1 represents a hydrogen atom, an unsubstituted alkyl group, an aryl group or an alkyl group that is substituted by one or more substituents selected from halogen atoms, alkoxy groups, alkylthio groups, cycloalkyl groups, cycloalkylthio groups and heterocyclic groups;
R2 represents an alkyl group substituted by a -COOR3 group, wherein R3 represents a hydrogen atom or an alkyl group that is substituted or not by one or more substituents selected from halogen atoms, cycloalkyl groups and heterocyclic groups.
R4 and R5, identical or different, represent an alkyl group that is substituted or not by one or more substituent selected from halogen atoms, cycloalkyl groups and heterocyclic groups;

- Acetyl-CoA carboxylase inhibitors such as GS-0976, ND-654, AC-8632, PF05175157, CP640186, gemcabene, MK-4074, and PF05175157;
- Adenosine A3 receptor agonists such as 2-(1-Hexynyl)-N-methyladenosine, Piclidenoson CF101 (IB-MECA), Namodenoson CF-102, 2-Cl-IB-MECA, CP-532,903, Inosine, LUF-6000, and MRS-3558;
- Aldosterone antagonists and mineralocorticoid receptor antagonists such as apararenone (MT 3995), amiloride, spironolactone, eplerenone, canrenone and potassium canrenoate, progesterone, drospirenone, gestodene, and benidipine;
- AMP activated protein kinase stimulators such as PXL-770, MB-11055, Debio-0930B, metformin, CNX-012, O-304, mangiferin calcium salt, eltrombopag, carotuximab, and imeglimin;
- Amylin receptor agonists and calcitonin receptor agonists such as KBP-042 and KBP-089;
- Antisense oligonucleotides targeting transforming growth factor beta 2 such as ASPH-0047, IMC-TR1 and ISTH-0047;
- Angiopoietin-related protein-3 inhibitors such as ARO-ANG3, IONIS-ANGGPTL3-LRx or AKCEA-ANGPTL3LRx, evinacumab, and ALN-ANG;
- Anti-LPS antibodies such as IMM-124-E;
- Apical sodium-codependent bile acid transporter inhibitors such as A-4250, volixibat, maralixibat formely SHP-625, GSK-2330672, elobixibat, and CJ-14199;
- Betaine anhydrous or RM-003;
- Bile acids such as obeticholic acid (OCA), ursodeoxycholic acid (UDCA), norursodeoxycholic acid, and ursodiol;
- Bioactive lipids such as 5-hydroxyeicosapentaenoic acid (15-HEPE, DS-102), unsaturated fatty acids such as 25 arachidonic acid, icosapentethyl ester, eicosapentaneoic acid, and docosahexaenoic acid;
- Cannabinoid CB1 receptor antagonists such as GRC-10801, MRI-1569, MRI-1867, DBPR-211, AM-6527 : AM-6545, NESS-11-SM, CXB-029, GCC-2680, TM-38837, Org-50189, PF-514273, BMS-812204, ZYO-1, AZD-2207, AZD-1175, otenabant, ibipinabant, surinabant, rimonabant, drinabant, SLV-326, V-24343, and O-2093;
- Cannabinoid CB2 receptor mimetics such as anabasum (Resunab, JKT-101);
- Dual cannabinoid CB1 receptor/iNOS inhibitor;
- Caspase inhibitors such as emricasan, belnacasan, nivocasan, IDN-7314, F-573, VX-166, YJP-60107, MX-1122, IDN-6734, TLC-144, SB-234470, IDN-1965, VX-799, SDZ-220-976, and L-709049;
- Cathepsin inhibitors such as VBY-376, VBY-825, VBY-036, VBY-129, VBY-285, Org-219517, LY3000328, RG-7236, and BF/PC-18;

- CCR antagonists such as cenicriviroc (CCR2/5 antagonist), PG-092, RAP-310, INCB-10820, RAP-103, PF-04634817, and CCX-872;
- CCR3 chemokine modulators and eotaxin 2 ligand inhibitors;
- Diacylglycerol-O-acyltransferase (DGAT) inhibitors such as IONIS-DGAT2Rx (formely ISIS-DGAT2Rx), LY-3202328, BH-03004, KR-69530, OT-13540, AZD-7687, and ABT-046;
- Dipeptidyl peptidase IV (DPP4) inhibitors such as evogliptin, vidagliptin, fotagliptin, alogliptin, saxagliptin, tilogliptin, anagliptin, sitagliptin, retagliptin, melogliptin, gosogliptin, trelagliptin, teneligliptin, dutogliptin, linagliptin, gemigliptin, yogliptin, betagliptin, imigliptin, omarigliptin, vidagliptin, and denagliptin;
- Insulin ligand and insulin receptor agonists;
- Insulin sensitizer and MCH receptor-1 antagonists;
- Dual NOX (NADPH oxidase) 1&4 inhibitors such as GKT-831 (2-(2-chlorophenyl)-4-[3-(dimethylamino) phenyl]-5-methyl-1H-pyrazolo[4,3-c]pyridine-3,6(2H,5H)-dione), formely GKT137831, and GKT-901;
- Extracellular matrix protein modulators such as CNX-024, CNX-025, and SB-030.
- Stearoyl CoA desaturase-1 inhibitors/fatty acid bile acid conjugates (FABAC);
- Farnesoid X receptor (FXR) agonists such as obeticholic acid (OCA), GS-9674, LJN-452, EDP-305, AKN-083, INT-767, GNF-5120, LY2562175, INV-33, NTX-023-1, EP-024297, Px-103, and SR-45023;
- Fatty acids such as omega-3 fatty acids, Omacor, MF4637, fish oils, and poly unsatured fatty acids (efamax, optiEPA);
- Fatty Acid Synthase (FAS) inhibitors such as TVB-2640; TVB-3199, TVB-3693BZL-101, 2-octadecynoic acid, MDX-2, Fasnall, MT-061, G28UCM, MG-28, HS-160, GSK-2194069, KD-023, cilostazol; and the following compounds:

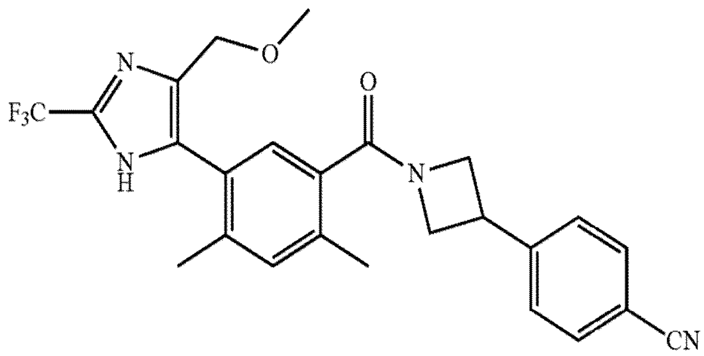

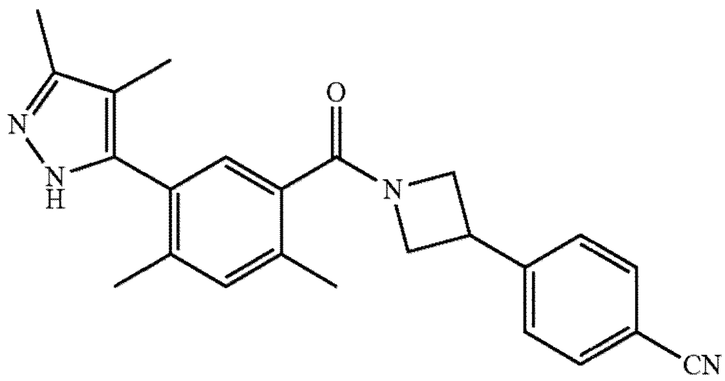

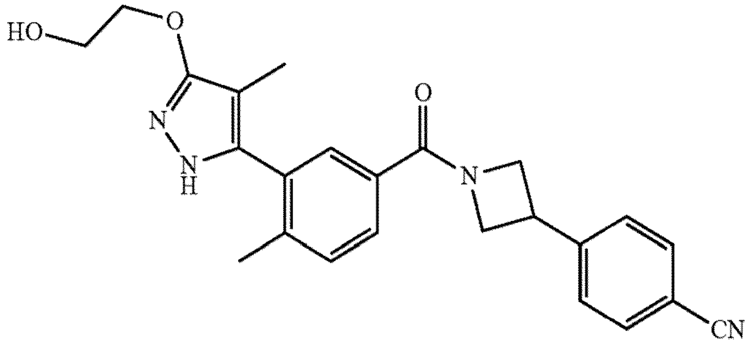

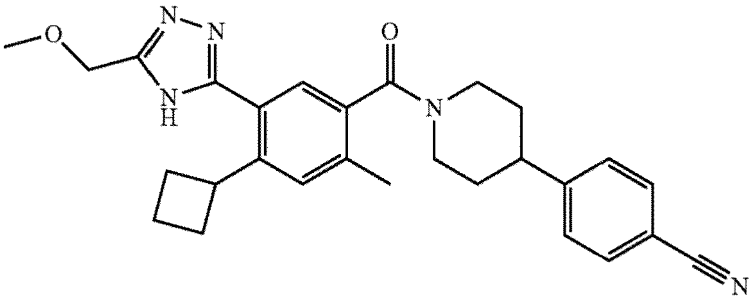

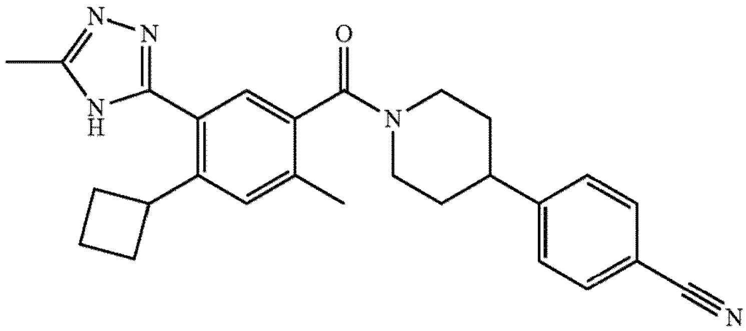

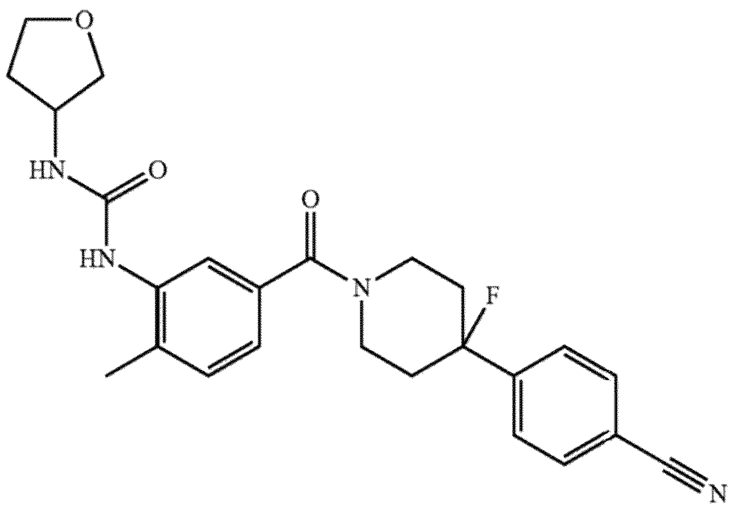

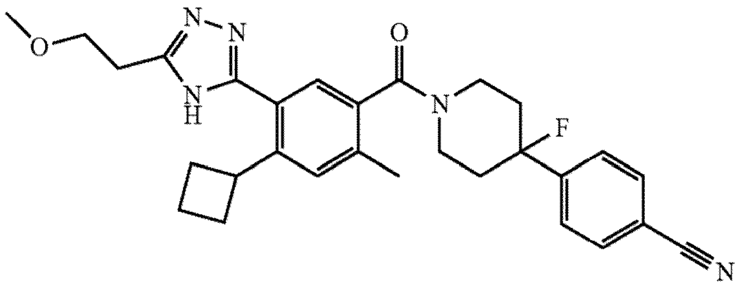

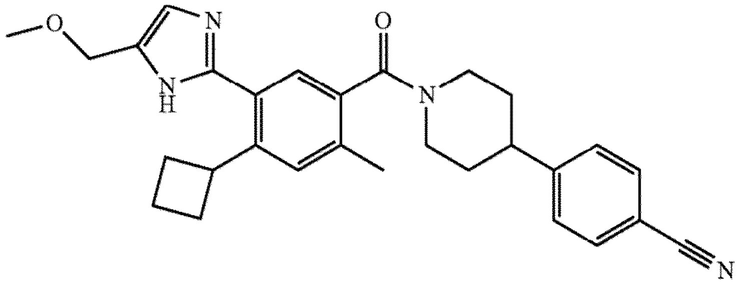

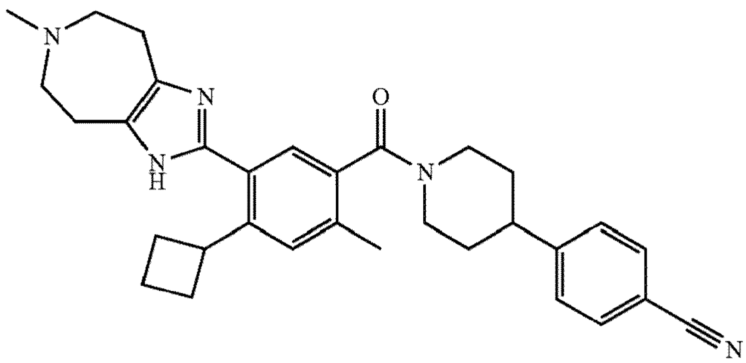

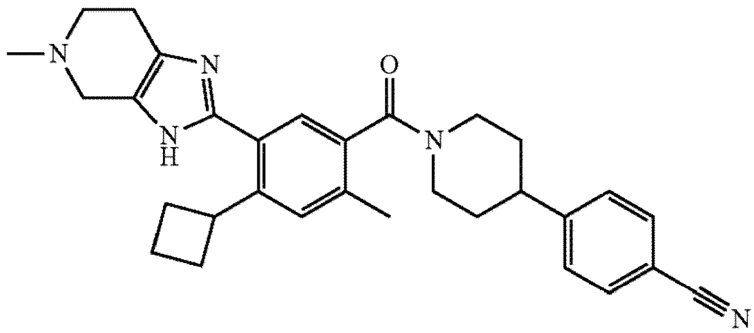

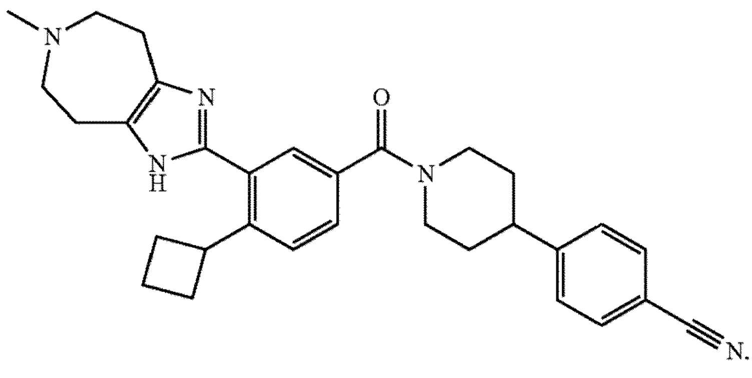
;

- Fibroblast Growth Factor 19 (FGF-19) receptor ligand or functional engineered variant of FGF-19;
- engineered Fibroblast Growth Factor 19 (FGF-19) analogues such as NGM-282;
- Fibroblast Growth Factor 21 (FGF-21) agonists such as PEG-FGF21 (formely BMS-986036), YH-25348, BMS-986171, YH-25723, LY-3025876, and NNC-0194-0499;
- Galectin 3 inhibitors such as GR-MD-02, TD-139, ANG-4021, Galectin-3C, LJPC-201, TFD-100, GR-MD-03, GR-MD-04, GM-MD-01, GM-CT-01, GM-CT-02, Gal-100, and Gal-200;
- Glucagon-like peptide-1 (GLP-1) analogs such as semaglutide, liraglutide, exenatide, albiglutide, dulaglutide, lixisenatide, loxenatide, efpeglenatide, taspoglutide, MKC-253, DLP-205, and ORMD-0901;
- Glucagon-like peptide-1 (GLP-1) receptor agonists like LY-3305677, and long-acting Oxyntomodulin;
- G-protein coupled receptor (GPCR) modulators;
- CNX-023;
- G-protein coupled receptor 84 antagonist (GPR84 antagonist);
- connective tissue growth factor ligand inhibitor and Free fatty acid receptor 1 agonist (FFAR1 agonist) such as PBI-4050,

PBI-4265, PBI-4283, and PBI-4299;

- Growth hormone;
- Hedgehog cell-signalling pathway inhibitors such as vismodegib, TAK-441, IPI-926, saridegib, sonidegib/erismodegib, BMS-833923/XL139, PF-04449913, taladegib/LY2940680, ETS-2400, SHR-1539, and CUR61414;
- Ileal sodium bile acid cotransporter inhibitors such as A-4250, GSK-2330672, volixibat, CJ-15 14199, and elobixibat;
- Immunomodulators such as PBI-4050, PBI-4265, PBI-4283, PBI-4299 and AIC-649;
- Insulin sensitizers and MCH receptor-1 antagonists such as MSDC-0602k, MSDC-0602, CSTI-100 and AMRI;
- Integrin inhibitors; integrin inhibitors of Pliant Therapeutic, integrin inhibitors of Indalo Therapeutics, integrin inhibitors of St Louis University, ProAgio, and GSK-3008348;
- Ketohexokinase inhibitors such as JNJ-28165722, JNJ-42065426; JNJ-42152981, JNJ-42740815, JNJ-42740828, and PF-06835919;
- Leukotriene (LT)/Phosphodiesterase (PDE)/Lipoxygenase (LO) inhibitors such as tipelukast (formely MN-001), tomelukast, sulukast, masilukast, zafirlukast, pranlukast, montelukast, gemilukast, verlukast, aklukast, pobilikast, cinalukast, and iralukast;
- Lysyl oxidase homolog 2 inhibitors like Rappaport, InterMune, Pharmaxis, AB-0023, Simtuzumab, PXS-5382A, and PXS-5338;
- Macrolides such as solithromycin, azithromycin, and erythromycin;
- Macrophage mannose receptor modulators such as AB-0023, MT-1001, [18F]FB18mHSA, Xemys, technetium Tc 99m tilmanocept, and CDX-1307;
- Methyl CpG binding protein 2 modulator and transglutaminase inhibitors such as cysteamine, EC Cysteamine, enteric-coated cysteamine bitartrate, cysteamine bitartrate (enteric-coated), Bennu, cysteamine bitartrate (enteric-coated), Raptor, cysteamine bitartrate, DR Cysteamine, delayed release enteric coated cysteamine bitartrate, mercaptamine, mercaptamine (enteric-coated), Bennu, mercaptamine (enteric-coated), Raptor, RP-103, RP-104, PROCYSBI, and mercaptamine (enteric-coated);
- miRNA antagonists like RG-125 (formely AZD4076), RGLS-5040, RG-101, MGN-5804, and MRG-201;
- Metalloproteinase 9 (MMP9) stimulator like MMP9 stimulator of Elastomic Ab;
- Mitochondrial carrier family inhibitors and Mitochondrial phosphate carrier protein inhibitors such as TRO-19622, Trophos, olesoxime, RG-6083, and RO-7090919;
- Myeloperoxidase inhibitors such as PF-06667272;
- Monoclonal antibodies such as bertilimumab, NGM-313, IL-20 targeting mAbs, fresolimumab (antiTGF$\beta$) (formely GC1008), timolumab (formely BTT-1023), namacizumab, omalizumab, ranibizumab, bevacizumab, lebrikizumab, epratuzumab, felvizumab, matuzumab, monalizumab, reslizumab, and inebilizumab;
- Monoclonal antibodies such as anti-IL20 mAbs, anti-TGF$\beta$ antibodies, anti-CD3 antibodies, anti-LOXL2 antibodies and anti-TNF antibodies;
- mTOR modulators such as MSDC-0602, AAV gene therapy co-administered with SVP-sirolimus;
- NAD-dependent deacetylase sirtuin stimulators, PDE 5 inhibitors such as NS-0200;
- NF-kappa B inhibitors such as LC-280126;
- Nicotinic acid such as Niacin and Vitamin B3;
- Nicotinic Acid Receptor (GPR109) Agonists such as ARI-3037MO, MMF, LUF 6283, acifran, IBC 293,

MK-1903, GSK256073, MK-6892, MK-0354, SLx-4090, lomitapide, lexibulin,
- apabetalone, acifran, laropiprant, daporinad, anacetrapib, INCB-19602, ST-07-02, lomefloxacin, Niacin, and controlled release/laropiprant;
- nitazoxanide (NTZ), its active metabolite tizoxanide (TZ) or other prodrugs of TZ such as RM-5061;
- non-steroid anti-inflammatory drugs (NSAIDs) such as F-351, salicylates (aspirin), acetaminophen, propionic acid derivatives (ibuprofen, naproxen), acetic acid derivatives (indomethacin, diclofenac), enolic acid derivatives (piroxicam, phenylbutazone), anthranilic acid derivatives (meclofenalmic acid, flufenamic acid), selective COX-2 inhibitors (celecoxib, parecoxib), and sulfonanilides (nimesulide);
- nuclear receptor ligands such as DUR-928 (formely DV 928);
- P2Y13 protein agonists such as CER-209;
- PDGFR modulators such as BOT-501 and BOT-191;
- Phenylalanine hydroxylase stimulators such as pegvaliase, sapropterin, AAV-PAH, CDX-6114, sepiapterin, RMN-168, ALTU-236, ETX-101, HepaStem, rolipram, and alprostadil;
- Protease-activated receptor (PAR)-2 antagonists such as PZ-235, and NP-003;
- Protein kinase modulators such as CNX-014, MB-11055, ALF-1, mangiferin, amlexanox, GS-444217, REG-101, and valine;
- PPAR alpha agonists such as fenofibrate, ciprofibrate, pemafibrate, gemfibrozil, clofibrate, binifibrate, clinofibrate, clofibric acid, nicofibrate, pirifibrate, plafibride, ronifibrate, theofibrate, tocofibrate, and SR10171;
- PPAR gamma agonists such as pioglitazone, deuterated pioglitazone, rosiglitazone, efatutazone, ATx08-001, OMS-405, CHS-131, THR-0921, SER-150-DN, KDT-501, GED-0507-34-Levo, CLC-3001, and ALL-4;
- PPAR delta agonists such as GW501516 (Endurabol or ({4-[({4-methyl-2-[4-(trifluoromethyl)phenyl]-1,3-thiazol-5-yl}methyl)sulfanyl]-2-methylphenoxy}acetic acid)), MBX8025 (Seladelpar or {2-methyl-4-[5-methyl-2-(4-trifluoromethyl- phenyl)-2H-[l,2,3]triazol-4-ylmethylsylfanyl]-phenoxy}-acetic acid), GW0742 ([4-[[[2-[3-fluoro-4-(trifluoromethyl)phenyl]-4-methyl-5-thiazolyl]methyl]thio]-2-methyl phenoxy]acetic acid), L165041, HPP-593, and NCP-1046;
- PPAR alpha/gamma dual agonists (also named glitazars) such as saroglitazar, aleglitazar, muraglitazar, tesaglitazar, and DSP-8658;
- PPAR alpha/delta dual agonists such as elafibranor, and T913659;
- PPAR gamma/delta dual agonists such as conjugated linoleic acid (CLA), and T3D-959;
- PPAR alpha/gamma/delta pan agonists or PPARpan agonists such as IVA337, TTA (tetradecylthioacetic acid), bavachinin, GW4148, GW9135, bezafibrate, lobeglitazone, and CS038;
- Prebiotic fibers;
- Probiotics;
- Pregnane X receptors such as rifampicin;
- Rho-associated protein kinase 2 (ROCK2) inhibitors such as KD-025, TRX-101, BA-1049, LYC-53976, INS-117548, and RKI-1447;
- signal-regulating kinase 1 (ASK1) inhibitors such as GS-4997;
- Sodium-glucose transport (SGLT) 2 inhibitors such as remogliflozin, dapagliflozin, empagliflozin, ertugliflozin, sotagliflozin, ipragliflozin, tianagliflozin, canagliflozin, tofogliflozin, janagliflozin, bexagliflozin, luseogliflozin, sergliflozin, HEC-44616, AST-1935, and PLD-101.
- stearoyl CoA desaturase-1 inhibitors/fatty acid bile acid conjugates such as aramchol, GRC-9332, steamchol, TSN-2998, GSK-1940029, and XEN-801;
- thyroid receptor $\beta$ (THR $\beta$) agonists such as VK-2809, MGL-3196, MGL-3745, SKL-14763, sobetirome, BCT-304, ZYT-1, MB-07811, and eprotirome;
- Toll Like Receptor 4 (TLR-4) antagonists such as naltrexone, JKB-121, M-62812, resatorvid, dendrophilin, CS-4771, AyuV-1, AyuV-25, NI-0101, EDA-HPVE7, and eritoran;
- Tyrosine kinase receptor (RTK) modulators such as CNX-025 and KBP-7018;
- Urate anion exchanger 1 inhibitors and xanthine oxidase inhibitors such as lesinurad, RLBN-1001, verinurad, KUX-1151, and lesinurad + allopurinol;
- Vascular adhesion protein-1 (VAP-1) inhibitors such as PXS-4728A, CP-664511, PRX-167700, ASP-8232, RTU-1096, RTU-007, and BTT-1023;
- Vitamin D receptor (VDR) agonists such as calciferol, alfacalcidol, 1,25-dihydroxyvitamin D3, Vitamin D2, Vitamin D3, calcitriol, Vitamin D4, Vitamin D5, dihydrotachysterol, calcipotriol, tacalcitol 1,24- dihydroxyvitamin D3, and paricalcitol; and
- Vitamin E and isoforms; vitamin E combined with vitamin C and atorvastatin.

Other anti-NASH agents include KB-GE-001, NGM-386, NGM-395, NC-10, icosabutate, NC-101, NAIA-101 colesevelam, and PRC-4016. Other anti-fibrotic agents include HEC-585, INV-240, RNAi therapeutic (Silence Therapeutics), SAMiRNA program (Bioneer Corp), pirfenidone, receptor tyrosine kinase inhibitors (RTKIs) such as Nintedanib, Sorafenib and other RTKIs, , angiotensin II (AT1) receptor blockers, , CTGF inhibitor, and any antifibrotic compound susceptible to interfere with the TGFβ and BMP-activated pathways including activators of the latent TGFβ complex such as MMP2, MMP9, THBS1 or cell-surface integrins, TGFβ receptors type I (TGFBRI) or type II (TGFBRII) and their ligands such as TGFβ, Activin, inhibin, Nodal, anti-Müllerian hormone, GDFs or BMPs, auxiliary co-receptors (also known as type III receptors), or components of the SMAD-dependent canonical pathway including regulatory or inhibitory SMAD proteins, or members of the SMAD-independent or non-canonical pathways including various branches of MAPK signaling, TAK1, Rho-like GTPase signaling pathways, phosphatidylinositol-3 kinase/AKT pathways, TGFβ-induced EMT process, or canonical and non-canonical Hedgehog signaling pathways including Hh ligands or target genes, or any members of the WNT, or Notch pathways which are susceptible to influence TGFβ.

In a particular embodiment, the anti-NASH agent is selected from a FXR agonist, a semicarbazide-sensitive amine oxidase (SSAO) inhibitor, a thyroid hormone receptor (THR) beta agonist and a GLP1 agonist/ASK-1 inhibitor. In a particular embodiment, the anti-NASH agent is selected from TERN-101 (6-{4-[5-Cyclopropyl-3-(2,6-dichlorophenyl)-isoxazol-4-ylmethoxy]-piperidin-1-yl}-1-methyl-1H-indole-3 carboxylic acid), TERN-201, TERN-501 and TERN-301.

In a particular embodiment, the anti-NASH agent is selected from ambrisentan, BMS-963272, BMS-986036, BMS-986251, BMS-986263, cenicriviroc, dapagliflozin, dulaglutide, elafibranor, empagliflozin, fenofibrate, HepaStem, lanifibranor, liraglutide, LYS006, MET409, MET642, MGL-3196, obeticholic acid, orlistat (Xenical), pioglitazone, resmetirom, rosiglitazone, saroglitazar magnesium, seladelpar, semaglutide, sitagliptin, TERN-101, TERN-201 and tropifexor (LJN452).

**EXAMPLES**

We assessed the clinical performance metrics of NIS4 (miR-34a-5p, a2M, YKL-40, HbA1c) either alone or in combination with other NITs: Fibrosis 4 (FIB4) and NAFLD Fibrosis Score (NFS), in patients characterized as T2D and with at-risk of NASH (NAS ≥ 4 and F ≥ 2).

1. Patients characteristics

RESOLVE-IT cohort (NCT02704403)

Patients were recruited at global centers (US, Latin America, Central America, and Europe). A liver biopsy was collected for all patients, and all biopsy samples were centrally read by an expert pathologist. Blood samples were collected prior to intervention for standard haematological and biochemical and biomolecular analyses. NASH diagnosed as steatohepatitis has been evaluated by a centrally-read liver biopsy taken within 6 months prior to randomization with at least a score of 1 in each component of the NAS score (steatosis scored 0-3, ballooning degeneration scored 0-2, and lobular inflammation scored 0-3), NAS ≥4 and fibrosis stage of 1 or greater and below 4, according to the NASH CRN fibrosis staging system.

For patients with type 2 diabetes, glycemia must be controlled.

Haematology and biochemical analyses were performed by the central laboratory of RESOLVE-IT trials (BARC-Europe, Ghent, Belgium) under fasting conditions.

Fibrosis-4 [FIB-4; Age, AST, ALT, Platelets], NAFLD activity score [NFS; Age, BMI, IGF/Diabetes Status, AST, ALT, Platelets, Albumin] and NIS4 [miR-34a-5p, alpha-2-macroglobulin, YKL-40, and hemoglobin A1c] were calculated according to the literature (Sterling R.K. et al, Hepatology, 2006; 43:1317-1325; Angulo P. et al, Hepatology, 2007;45:846-854, WO2017167934 and Harrison S.A, et al, The Lancet Gastroenterology and Hepatology, 2020; 5(11):P970-985).

Patients in the cohort presented with one or more risk factors for NASH and had corresponding NIT data: NIS4 (miR-34a-5p, a2M, YKL-40, HbA1c), Fibrosis-4 (FIB-4) and NAFLD Fibrosis score (NFS) data were used to identify patients with at-risk NASH.

The 467 patients were divided into 2 subgroups with 276 non-T2D and 191 T2D with a prevalence for at-risk NASH 47%, and 65%, respectively (Table1).

Table 1: Demographic data for patients.

| Variable | nonT2D patients | Type 2 Diabetic (T2D) patients | P value (Chi2, Wilcoxon) |
|---|---|---|---|
| number of patients | 276 | 191 | |

(continued)

| Variable | nonT2D patients | Type 2 Diabetic (T2D) patients | P value (Chi2, Wilcoxon) |
|---|---|---|---|
| Dyslipidemia | 39% ; n=109 | 63% ; n=121 | 3,97E-07 |
| Hypertension | 52% ; n=144 | 73% ; n=139 | 7,49E-06 |
| Number of comorbidities (T2D, dyslipidemia, hypertension, obesity) | 0: 11% ; n=29 1: 32% ; n=88 2: 38%; n=104 3: 20% ; n=55 4: 0%; n=0 | 0: 0% ; n=0 1: 4% ; n=8 2: 19% ; n=36 3: 37% ; n=71 4: 40% ; n=76 | 2,47E-06 |
| Gender (male) | 49% ; n=135 | 41% ; n=78 | 8,50E-02 |
| BMI≥30 | 75% ; n=208 | 76% ; n=146 | 7,89E-01 |
| At-risk NASH | 47% ; n=131 | 65% ; n=125 | 1,24E-04 |
| Advanced fibrosis | 24% ; n=66 | 41% ; n=78 | 9,87E-05 |
| Fibrosis stage | 1.61 ± 1.04 | 2.06 ± 1 | 6,82E-06 |
| NAS score | 4.45 ± 1.8 | 5.07 ± 1.41 | 8,54E-04 |
| Age | 53.24 ± 12.04 | 56.66 ± 10.83 | 2,30E-03 |
| BMI | 34.13 ± 5.93 | 34.06 ± 6.23 | 9,15E-01 |
| Alat | 65.57 ± 47.79 | 63.95 ± 42.78 | 7,48E-01 |
| Asat | 46.53 ± 31.5 | 45.94 ± 28.01 | 6,01E-01 |
| HOMA-IR | 6.37 ± 4.31 | 10.72 ± 8.87 | 1,77E-10 |
| Fasting plasma glucose | 5.35 ± 0.82 | 7.25 ±2.19 | 4,02E-31 |
| FIB4 | 1.42 ± 0.99 | 1.46 ± 0.88 | 3,69E-01 |
| NFS | -1.93 ± 1.47 | -0.71 ± 1.32 | 4,75E-21 |
| FibroScan | 11.44 ± 8.73 | 12.72 ± 8.51 | 3,44E-03 |
| NIS4 | 0.41 ± 0.25 | 0.61 ± 0.25 | 1,96E-16 |
| Hsa-miR-34a-5p | 31.8 ± 1.05 | 31.48 ± 0.99 | 1,26E-03 |
| YKL-40 | 82.44 ± 128.64 | 103.67 ± 127.09 | 1,91E-03 |
| A2M | 2.27 ± 0.89 | 2.49 ± 0.86 | 2,18E-03 |
| HbA1c | 5.68 ± 0.56 | 6.91 ± 0.94 | 1,38E-43 |

2. Results

The results show that 47% of non-T2D patients had at-risk NASH, while it was 65% for T2D patients. Patients with at-risk NASH have worse biochemical features as compared to patients without at-risk NASH, including elevated ALT and AST, worse glycemic control (HOMA-IR), and higher mean non-invasive scores (FIB-4, NFS & NIS4) (Table 1).

Data were analyzed using NIS4, FIB4 and NFS for single or double test combinations (Figure 1) and established clinical cutoffs of each test. Test metrics were quantified per diagnostic algorithm including the overall diagnostic accuracy to identify patients with at-risk NASH. Accuracy was defined as (True Positives + True Negatives) / (True Positives + False Positives + True Negatives + False Negatives). Sensitivity was defined as (True Positives) / (True Positives + False Negatives). Specificity was defined as (True Negatives)/ (True Negatives + False Positives). The overall diagnostic performance of each algorithm was also calculated in terms of sensitivity (Sen) and specificity (Spe). The absolute difference between specificity and sensitivity (Abs.Diff) allowed the selection of the best performing assay to diagnose patients at risk NASH.

Data indicated that for the non-T2D populations, NIS4 alone provided the best performance (accuracy = 74%, sensitivity = 76%, specificity = 72%, and absolute difference < 5) for identifying patients for at-risk NASH by selecting the low cutoff (0.36) for at-risk NASH. Accuracy (74%) was close to specificity (72%) and sensitivity (76%). The absolute difference between sensitivity and specificity was the lowest with NIS4, meaning that NIS4 performed better than FIB4 and NAFLD fibrosis score in non-diabetic patients.

In T2D patients, the use of NIS4 alone resulted in a highly sensitive diagnostic of patients with at risk NASH (91%) but showed a low specificity (41%). Interestingly, the sequential use of NIS4 together with another blood-based test NAFLD Fibrosis score (NFS) test provided an overall more balanced performance (accuracy = 73%, sensitivity = 77%, specificity = 65%, and absolute difference =12) for identification of at-risk NASH in T2D patients with a comparable accuracy to NIS4 in non T2D population: 73% in diabetic population versus 74% in non-T2D population. The order of diagnostic assay application (NIS4 then NFS versus NFS then NIS4) did not have any impact on diagnostic performances. By contrast, the sequential use of NIS 4 and FIB4 or FIB4 and NFS resulted in higher absolute difference between sensitivity and specificity both in T2D and non-T2D populations.

Sensitivity and specificity are independent characteristics determined by the established cutoff of the test. To manage diagnosis, the most accurate diagnostic procedure should have the potential to completely discriminate subjects with (sensitivity) and without disease (specificity). It means that sensitivity and sensitivity must be as high as possible and balanced.

It is herein shown that in T2D patients, a metabolic disorder associated to NASH, the best diagnostic performance was obtained by combining NIS4 and NFS.

## Claims

1. A method for the diagnosis of at-risk nonalcoholic steatohepatitis (NASH) in a human patient with a metabolic disorder, comprising:

   (i) calculating a nonalcoholic fatty liver disease score (NFS) from the age, body-mass index, aspartate amino transferase (AST)/ alanine aminotransferase (ALT) ratio, platelet count and hyperglycemia status of said patient; and
   (ii) calculating a nonalcoholic steatohepatitis score (NIS4) for said patient, based on the level of hsa-miR-34a-5p, alpha-2 macroglobulin (A2M), YKL-40 and glycated haemoglobin (HbA1c) measured in a blood or blood-derived sample of said patient;

   and determining the presence or absence of at-risk NASH in said patient based on said scores;
   wherein the NFS test is implemented before or after the NIS4 test.

2. The method according to claim 1, wherein the calculated NFS is compared to a NFS low cutoff value and the calculated NIS4 is compared to a NIS4 low cutoff value.

3. The method according to claim 2, wherein if the calculated NFS is lower than said NFS low cutoff value or if the calculated NIS4 is lower than said NIS4 low cutoff value, then the patient is diagnosed as not having at-risk NASH.

4. The method according to claim 2, wherein if the calculated NFS is greater or equal to said NFS low cutoff value and if the calculated NIS4 is greater or equal to said NIS 4low cutoff value, then the patient is diagnosed as having at-risk NASH.

5. An anti-NASH or anti-fibrotic agent for use in a method for the treatment of NASH in a patient in need thereof diagnosed as having at-risk NASH according to the method of any one of claims 1 to 4, wherein the agent is selected in the group consisting of ambrisentan, BMS-963272, BMS-986036, BMS-986251, BMS-986263, cenicriviroc, dapagliflozin, dulaglutide, empagliflozin, fenofibrate, HepaStem, lanifibranor, liraglutide, LYS006, MET409, MET642, MGL-3196, obeticholic acid, orlistat (Xenical), pioglitazone, resmetirom, rosiglitazone, saroglitazar magnesium, seladelpar, semaglutide, sitagliptin, TERN-101, TERN-201 and tropifexor (LJN452).

6. An anti-NASH or anti-fibrotic agent for use in a method for slowing down or stopping the progression of NASH in a patient in need thereof diagnosed as having at-risk NASH according to the method of any one of claims 1 to 4, wherein the agent is selected in the group consisting of ambrisentan, BMS-963272, BMS-986036, BMS-986251, BMS-986263, cenicriviroc, dapagliflozin, dulaglutide, empagliflozin, fenofibrate, HepaStem, lanifibranor, liraglutide, LYS006, MET409, MET642, MGL-3196, obeticholic acid, orlistat (Xenical), pioglitazone, resmetirom, rosiglitazone, saroglitazar magnesium, seladelpar, semaglutide, sitagliptin, TERN-101, TERN-201 and tropifexor (LJN452).

7. The anti-NASH or anti-fibrotic agent for use according to any one of claims 5 or 6, wherein the agent is administered to the patient if the calculated NFS and the calculated NIS4 are above the NFS low cutoff and the NIS4 low cutoff, respectively.

## Patentansprüche

1. Verfahren zur Diagnose einer Risiko-Nichtalkoholischen-Steatohepatitis (NASH) bei einem menschlichen Patienten mit einer Stoffwechselstörung, umfassend:

   (i) Berechnung eines Nichtalkoholische-Fettleber-Erkrankungs-Scores (Nonalcoholic Fatty Liver Disease Score, NFS) anhand von Alter, Body-Mass-Index, Aspartat-Aminotransferase (AST)/Alanin-Aminotransferase(ALT)-Quotient, Thrombozytenzahl und Hyperglykämiestatus dieses Patienten; und
   (ii) Berechnung eines Nichtalkoholischen-Steatohepatitis-Scores (Nonalcoholic Steatohepatitis Score, NIS4) für diesen Patienten basierend auf den in einer Blut- oder Blutprobe dieses Patienten gemessenen Konzentrationen von hsa-miR-34a-5p, Alpha-2-Makroglobulin (A2M), YKL-40 und glykiertem Hämoglobin (HbA1c);

   und Bestimmung des Vorliegens oder Nichtvorliegens einer Risiko-NASH bei diesem Patienten anhand dieser Scores;
   wobei der NFS-Test vor oder nach dem NIS4-Test durchgeführt wird.

2. Das Verfahren nach Anspruch 1, wobei der berechnete NFS-Wert mit einem unteren NFS-Grenzwert (cut-off-value) verglichen werden und der berechnete NIS4-Wert mit einem unteren NIS4-Grenzwert (cut-off-value) verglichen wird.

3. Das Verfahren nach Anspruch 2, wobei, wenn der berechnete NFS-Wert unterhalb dieses unteren NFS-Grenzwerts (cut-off-value) liegt oder wenn der berechnete NIS4-Wert unterhalb dieses unteren NIS4-Grenzwerts (cut-off-value) liegt, der Patient als nicht an Risiko-NASH erkrankt diagnostiziert wird.

4. Das Verfahren nach Anspruch 2, wobei, wenn der berechnete NFS-Wert größer oder gleich diesem unteren NFS-Grenzwert (cut-off-value) und wenn der berechnete NIS4-Wert größer oder gleich diesem unteren NIS4-Grenzwert (cut-off-value) ist, der Patient als an Risiko-NASH erkrankt diagnostiziert wird.

5. Anti-NASH- oder antifibrotisches Mittel zur Verwendung in einem Verfahren zur Behandlung von NASH bei einem Patienten, der dies benötigt, der gemäß dem Verfahren eines der Ansprüche 1 bis 4 als an Risiko-NASH erkrankt diagnostiziert wurde, wobei das Mittel ausgewählt ist aus der Gruppe bestehend aus Ambrisentan, BMS-963272, BMS-986036, BMS-986251, BMS-986263, Cenicriviroc, Dapagliflozin, Dulaglutid, Empagliflozin, Fenofibrat, HepaStem, Lanifibranor, Liraglutid, LYS006, MET409, MET642, MGL-3196, Obeticholsäure, Orlistat (Xenical), Pioglitazon, Resmetirom, Rosiglitazon, Saroglitazar-Magnesium, Seladelpar, Semaglutid, Sitagliptin, TERN-101, TERN-201 und Tropifexor (LJN452).

6. Anti-NASH- oder antifibrotisches Mittel zur Verwendung in einem Verfahren zur Verlangsamung oder zum Stoppen des Fortschreitens von NASH bei einem Patienten, der dies benötigt, der gemäß dem Verfahren eines der Ansprüche 1 bis 4 als an Risiko-NASH erkrankt diagnostiziert wurde, wobei das Mittel ausgewählt ist aus der Gruppe bestehend aus Ambrisentan, BMS-963272, BMS-986036, BMS-986251, BMS-986263, Cenicriviroc, Dapagliflozin, Dulaglutid, Empagliflozin, Fenofibrat, HepaStem, Lanifibranor, Liraglutid, LYS006, MET409, MET642, MGL-3196, Obeticholsäure, Orlistat (Xenical), Pioglitazon, Resmetirom, Rosiglitazon, Saroglitazar-Magnesium, Seladelpar, Semaglutid, Sitagliptin, TERN-101, TERN-201 und Tropifexor (LJN452).

7. Das Anti-NASH- oder Antifibrotikum zur Verwendung nach einem der Ansprüche 5 oder 6, wobei das Mittel dem Patienten verabreicht wird, wenn der berechnete NFS-Wert und der berechnete NIS4-Wert jeweils über dem unteren Grenzwert des NFS-Werts (cut-off-value) bzw. dem unteren Grenzwert des NIS4-Werts (cut-off-value) liegen.

## Revendications

1. Méthode pour le diagnostic d’une stéato-hépatite non alcoolique (NASH) à risque chez un patient humain présentant un trouble métabolique, comprenant :

   (i) le calcul du score de maladie de stéatose hépatique non alcoolique (NFS) d’après l’âge, l’indice de masse corporelle, le rapport aspartate aminotransférase (AST) / alanine aminotransférase (ALT), le compte de plaquettes et le statut d’hyperglycémie dudit patient ; et
   (ii) le calcul d’un score de stéato-hépatite non alcoolique (NIS4) pour ledit patient, sur la base des niveaux de hsa-miR-34a-5p, d’alpha-2 macroglobuline (A2M), de YKL-40 et d’hémoglobine glyquée (HbA1c) mesurés dans un

échantillon de sang ou dérivé de sang dudit patient ;

et la détermination de la présence ou de l'absence d'une NASH à risque chez ledit patient sur la base desdits scores ;
dans laquelle le test de NFS est effectué avant ou après le test de NIS4.

2. Méthode selon la revendication 1, dans laquelle le NFS calculé est comparé à un seuil de coupure bas de NFS, et le NIS4 calculé est comparé à un seuil de coupure bas de NIS4.

3. Méthode selon la revendication 2, dans laquelle, si le NFS calculé est inférieur audit seuil de coupure bas de NFS ou si le NIS4 calculé est inférieur audit seuil de coupure bas de NIS4, alors le patient est diagnostiqué comme n'ayant pas de NASH à risque.

4. Méthode selon la revendication 2, dans laquelle, si le NFS calculé est supérieur ou égal audit seuil de coupure bas de NFS ou si le NIS4 calculé est supérieur ou égal audit seuil de coupure bas de NIS4, alors le patient est diagnostiqué comme ayant une NASH à risque.

5. Agent anti-NASH ou antifibrotique pour une utilisation dans une méthode pour le traitement d'une NASH chez un patient en ayant besoin, diagnostiqué comme ayant une NASH à risque conformément à la méthode de l'une quelconque des revendications 1 à 4, lequel agent est choisi dans le groupe constitué par l'ambrisentan, BMS-963272, BMS-986036, BMS-986251, BMS-986263, le cénicriviroc, la dapagliflozine, le dulaglutide, l'empagliflozine, le fénofibrate, HepaStem, le lanifibranor, le liraglutide, LYS006, MET409, MET642, MGL-3196, l'acide obéticholique, l'orlistat (Xenical), la pioglitazone, le resmétirom, la rosiglitazone, le saroglitazar magnésium, le séladelpar, le sémaglutide, la sitagliptine, TERN-101, TERN-201 et le tropifexor (LJN452).

6. Agent anti-NASH ou antifibrotique pour une utilisation dans une méthode pour ralentir ou stopper la progression d'une NASH chez un patient en ayant besoin, diagnostiqué comme ayant une NASH à risque conformément à la méthode de l'une quelconque des revendications 1 à 4, lequel agent est choisi dans le groupe constitué par l'ambrisentan, BMS-963272, BMS-986036, BMS-986251, BMS-986263, le cénicriviroc, la dapagliflozine, le dulaglutide, l'empagliflozine, le fénofibrate, HepaStem, le lanifibranor, le liraglutide, LYS006, MET409, MET642, MGL-3196, l'acide obéticholique, l'orlistat (Xenical), la pioglitazone, le resmétirom, la rosiglitazone, le saroglitazar magnésium, le séladelpar, le sémaglutide, la sitagliptine, TERN-101, TERN-201 et le tropifexor (LJN452).

7. Agent anti-NASH ou antifibrotique pour une utilisation selon l'une quelconque des revendications 5 et 6, lequel agent est administré au patient si le NFS calculé et le NIS4 calculé sont supérieurs respectivement au seuil de coupure bas de NFS et au seuil de coupure bas de NIS4.

**At risk NASH patients**

| | nonT2D | | | | | | T2D | | | | | |
|---|---|---|---|---|---|---|---|---|---|---|---|---|
| | n | At-risk prevalence | Acc | Sen | Spe | Abs. Diff | n | At-risk prevalence | Acc | Sen | Spe | Abs. Diff |
| NIS4 ≥ 0.36 | 276 | 47% | 74 | 76 | 72 | 5 | 191 | 65% | 74 | 91 | 41 | 50 |
| FIB4 ≥ 1.3 | 276 | 47% | 71 | 66 | 76 | 10 | 191 | 65% | 64 | 58 | 77 | 20 |
| NFS ≥ -1.455 | 276 | 47% | 64 | 53 | 75 | 23 | 191 | 65% | 66 | 82 | 35 | 48 |
| NIS4 ≥ 0.36 then FIB4 ≥ 1.3 | 276 | 47% | 72 | 57 | 86 | 29 | 191 | 65% | 65 | 54 | 86 | 32 |
| FIB4 ≥ 1.3 then NIS4 ≥ 0.36 | 276 | 47% | 72 | 57 | 86 | 29 | 191 | 65% | 65 | 54 | 86 | 32 |
| NIS4 ≥ 0.36 then NFS ≥ -1.455 | 276 | 47% | 68 | 40 | 92 | 52 | 191 | 65% | 73 | 77 | 65 | 12 |
| NFS ≥ -1.455 then NIS4 ≥ 0.36 | 276 | 47% | 68 | 40 | 92 | 52 | 191 | 65% | 73 | 77 | 65 | 12 |
| NFS ≥ -1.455 then FIB4 ≥ 1.3 | 276 | 47% | 67 | 44 | 88 | 44 | 191 | 65% | 62 | 54 | 77 | 24 |
| FIB4 ≥ 1.3 then NFS ≥ -1.455 | 276 | 47% | 67 | 44 | 88 | 44 | 191 | 65% | 62 | 54 | 77 | 24 |

**FIGURE 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017167934 A


### Non-patent literature cited in the description


- **CALDAZILLA S.H. et al.** *Int J Mol Sci*, 2016, vol. 17 (5), 774
- **TOMAH, S. et al.** *Clinical Diabetes and Endocrinology*, 2020, vol. 6, 9
- **KOGACHI SHANNON et al.** Noninvasive Evaluation for Nonalcoholic Fatty Liver Disease and Nonalcoholic Steatohepatitis. *CLINICAL THERAPEUTICS*, 10 February 2021, vol. 43, 455-472
- **GLAROS A.G. et al.** *J Clin Psychol*, 1988, vol. 44 (6), 1013-23
- **KLEINER, D.E et al.** *Hepatology*, June 2005, vol. 41 (6), 1313-21
- **ANGULO et al.** *Hepatology*, 2007, vol. 45, 846-854
- **HARRISON et al.** *The Lancet Gastroenterology and Hepatology*, 2020, vol. 5 (11), 970-985
- **STERLING R.K. et al.** *Hepatology*, 2006, vol. 43, 1317-1325
- **ANGULO P. et al.** *Hepatology*, 2007, vol. 45, 846-854
- **HARRISON S.A et al.** *The Lancet Gastroenterology and Hepatology*, 2020, vol. 5 (11), 970-985